# EUROPEAN PATENT SPECIFICATION

(11) **EP 1 888 085 B1**
(45) Date of publication and mention of the grant of the patent: **08.08.2012**
(21) Application number: 05757166.3
(22) Date of filing: 03.06.2005
(51) Int. Cl.: A61K 38/01

(54) **METHODS FOR PROVIDING PALLIATIVE CARE WITH PRODUCT R**
VERFAHREN FÜR DIE PALLIATIVE VERSORGUNG MIT PRODUCT R
PROCEDES OFFRANT DES SOINS PALLIATIFS A BASE DE PRODUCT R

(43) Date of publication of application: 20.02.2008
(73) Proprietor: OHR Pharmaceutical, Inc., New York, NY 10017 (US)
(72) Inventor: HIRSCHMAN, Shalom, Z., Riverdale, NY 10471 (US)
(74) Representative: Smithson, Robert Alan
(86) International application number: PCT/US2005/019634
(87) International publication number: WO 2006/132623

(56) References cited:
- US-A- 5 849 196
- US-A1- 2002 004 579
- US-A1- 2004 033 244
- US-A1- 2004 033 244
- LEVETT PAUL N ET AL: "Randomized, placebo-controlled trial of product R, a peptide-nucleic acid immunomodulator, in the treatment of adults infected with HIV." HIV CLINICAL TRIALS 2002 JUL-AUG, vol. 3, no. 4, July 2002 (2002-07), pages 272-278, XP007911557 ISSN: 1528-4336
- LAZZARINO DEBORAH A ET AL: "IL-8 and MCP-1 secretion is enhanced by the peptide-nucleic acid immunomodulator, Product R, in U937 cells and primary human monocytes" CYTOKINE, vol. 14, no. 4, 21 May 2001 (2001-05-21), pages 234-239, XP007911558 ISSN: 1043-4666
- D'OLIMPIO J.T. ET AL.: 'Anti-cachectic effects of a novel peptide nucleic acid: preliminary results of a phase I/II clinical trial' JOURNAL OF CLINICAL ONCOLOGY, 2004 AMERICAN SOCIETY OF CLINICAL ONCOLOGY ANNUAL MEETING PROCEEDINGS vol. 22, no. 14S, 2004, page 8087, XP008070819
- WANG W.: 'Technology evaluation: reticulose, advanced viral research' CURRENT OPINION IN MOLECULAR THERAPEUTICS vol. 5, no. 2, 2003, pages 186 - 191, XP008070813

### SEQUENCE LISTING

<110> Advanced Viral Research Corp.
<120> METHODS FOR PROVIDING PALLIATIVE CARE WITH AVR118
<130> 11530-061-228
<140> to be assigned
   <141>
<160> 2
<170> FastSEQ for Windows Version 4.0
<210> 1
   <211> 31
   <212> PRT
   <213> Bovine
<400> 1
   Lys Val Leu Pro Val Pro Gln Lys Ala Val Pro Tyr Pro Gln Arg Asp
    1               5                  10                  15
   Met Pro Ile Gln Ala Phe Leu Leu Tyr Gln Glu Pro Val Leu Gly
               20                  25                  30
<210> 2
   <211> 21
   <212> PRT
   <213> Bovine
<400> 2
   Gly Glu Ile Pro Asp Ala Gly Gly Arg Ile Val Asp Tyr Tyr Val Gly
    1              5                   10                  15
   Phe Ser Asp Ser Val
               20

## Description

### 1. FIELD OF THE INVENTION

The present invention relates to methods for using AVR118 (Product R), a peptide- nucleic acid composition, to provide palliative care, e.g., to treat cachexia, anorexia and/or improve quality of life, in patients with terminal diseases. The methods are applicable for patients suffering from end-stage cancers and other diseases.

### 2. BACKGROUND OF THE INVENTION

### 2.1. Palliative Care

Palliative care is any form of medical care or treatment that concentrates on reducing the severity of the symptoms of a disease or slows its progress rather than providing a cure. It is particularly important in diseases in which there is no hope of survival. It aims at improving quality of life, and particularly at reducing or eliminating pain. Patients with terminal diseases, such as an advanced cancer, suffer from a complex group of symptoms including, but not limited to, cachexia, anorexia, fatigue, weakness, and pain. These symptoms further affect the patient's mood and level of daily activities.

Cachexia is the name given to a generally weakened condition of the body or mind resulting from any debilitating chronic disease. Typical symptoms of cachexia include severe weight loss, depression, loss of appetite, anorexia and anemia. Cachexia is normally associated with neoplastic diseases, chronic infectious diseases or thyroiditis, and is a particular problem when associated with cancerous conditions. It has been estimated that more than 20% of all cancer deaths are associated with cachexia (Tisdale, 2002, Nat Rev Cancer 2:862-871). Cachexia is believed to be induced by the secretion of the cytokine TNF (Tracey et al, 1988, J. Exp. Med 167:1211-1227).

Nutritional supplements are often given in order to treat the symptoms of cachexia. This, however, often enhances the progress of the malignant tumor, and may shorten the survival time of the patient. Corticosteroids have been tested for cachexia and have resulted in improvements in patients, such as increased appetite, food intake, and general well-being, but they are ineffective for weight gain (Bruera et ah, 1998, CMAJ158:1717-26). Progestational drugs have been shown to increase appetite, weight gain and well-being; however these drugs are expensive and have adverse effects associated with them (Bruera et ah, 1998, CMAJ 158:1717-26). Other therapeutic agents being studied for use in cachexia are described in Inui, 2002, CA Cancer J Clin 52:72-91.

Reticulose<(R)> emerged as an antiviral product in the 1930's. While it was originally believed to be a product composed of peptone, peptides and nucleic acids, the precise composition remains unidentified. A method for preparing Reticulose<(R)> is provided in U.S. Patent No. 5,849,196. Reticulose<(R)> has demonstrated an ability to inhibit rapidly the course of several viral diseases. It is nontoxic, miscible with tissue fluids and blood sera and free from anaphylactogenic properties.

As taught by U.S. Patent No. 5,849,196, the components present in the conventional composition of Reticulose<(R)> that are greater than 15 kDa in molecular weight are more effective in treating viral diseases such as HIV, influenza virus, herpes simplex virus, etc., while the components having a molecular weight in a range of approximately 1 to 15 KDa function as phagocytosis inhibitors.

U.S. Patent Nos. 6,303,153 and 6,528,098, disclose the preparation of AVR118 (referred to herein and generally in the art as Product R), a composition derived from the same starting materials as used in preparing Reticulose , but distinct from Reticulose<(R)>. For example, material greater than 14 kDa molecular weight is removed when preparing Product R.

Product R is an immunomodulator with a favorable safety profile affecting the production of pro-inflammatory chemokines and cytokines, driving the immune response towards a normal state. In particular, Product R modulates the expression of chemokines and cytokines. Product R stimulates macrophages to produce pro-inflammatory cytokines and chemokines, including MCP-I, IL-8 and IL-6 (Lazzarino et al, 2001, Cytokine, 14:234-239; Lazzarino et al, 2000, Immunol. Lett. 74:189-195). On the other hand, in highly activated macrophages, Product R inhibits the synthesis of these pro- inflammatory chemokines. Product R may also increase levels of IL-2R. IL-2R can stimulate populations of both T and B cells.

Although considerable advances have occurred in the field of palliative care, palliative care still presents a formidable challenge to the physician. At present, no satisfactory treatment for cachexia has been established, and there is an increasing need for agents that alleviate the symptoms of cachexia.

### 3. SUMMARY OF THE INVENTION

The present invention provides methods for providing palliative care in a patient with a non-lymphocytic cancer which is not a basal cell carcinoma, comprising administering to said patient an amount of Product R effective to treat or prevent cachexia or improve quality of life, wherein within three weeks of said administering of Product R said patient has not and does not have chemotherapy or radiation therapy. In the methods and compositions of the invention, Product R comprises nucleotides and peptides having molecular weights not more than 14 KDa and substantially not more than 8 KDa, wherein said nucleotides and peptides are breakdown products of casein, peptone, RNA and serum albumin, and wherein said composition has a light absorption spectrum with typical absorption ratios of 2.0 (+-10%) at 260 nm/280 nm and 1.4 (+-10%) at 260 nm/230 nm. In a preferred embodiment, the patient is a human. In certain embodiments, the amount of Product R effective to treat or prevent cachexia and/or improve quality of life is in a range from about 0.5 microliters to about 100 microliters per kilogram of body weight per day, or from about 2.5 microliters to about 40 microliters per kilogram of body weight per day, or from about 10 microliters to about 25 microliters per kilogram of body weight per day. In certain embodiments of the invention, Product R is administered parenterally, topically or systemically.

The present invention also encompasses methods for providing palliative care, comprising administering to a patient with an end-stage non-lymphocytic cancer (1) an amount of Product R effective to treat or prevent cachexia and/or improve QOL and (2) an effective amount of another medicament. In certain embodiments, the other medicament is an analgesic or adjuvant drug.

The present invention also comprises pharmaceutical compositions and kits comprising 1) an amount of Product R effective to treat or prevent cachexia and/or improve QOL; 2) an analgesic or adjuvant drug; and 3) a pharmaceutically acceptable carrier.

Analgesics useful in the methods and compositions of the invention include, but are not limited to, nonopioid analgesics, such as acetaminophen and nonsteroidal antiinflammatory drugs (NSAIDs); opioid analgesics, such as codeine, hydromorphone, morphine, oxycodone, fentanyl, and methadone. Adjuvant drugs useful in the methods of the invention include, but are not limited to, tricyclic antidepressants, corticosterioids, anticonvulsants, biphosphonates, oral local anesthetics, pyschostimulants and muscle relaxants. Other medicaments useful in the methods and compositions of the invention include, but are not limited to, a vitamin (e.g., Vitamins A, C, D, E, K5 and the eight B vitamins-biotin, folate, niacin, pantothenic acid, riboflavin, thiamin, vitamin B6, and vitamin B 12), an herbal supplement (e.g., chondroitin), a sleep aid (e.g., Zolpidem Tartrate (Ambien<(R)>)), a lipid lowering agent (e.g., Atorvastatin Calcium (Lipitor<(R)>)), an antiasthmatic agent (e.g., theophylline), an anti-constipation agent (e.g., Senokot<(R)> (Purdue Products L.P., Stamford, Connecticut)), an anti-heartburn agent (e.g., Nexium, Pepcid, Prevacid, Prilosec, Tagamet, Zantac, etc.), or an anti-hypertensive agent.

### 4. DETAILED DESCRIPTION OF THE INVENTION

The inventors have discovered that Product R can treat cachexia and improve weight gain in patients with cancer, particularly end-stage cancer. The present invention provides methods and compositions for providing palliative care with Product R. Product R can be used provide palliative care in patients with a variety of diseases and improve their quality of life in their final days. Without being bound by any theory, Product R is believed to modulate the immune system.

Product R (Advanced Viral Research Corp., Yonkers, NY) is a composition comprising the breakdown products of casein, peptone, RNA, and serum albumin. The manufacturing process, composition, and the chemical and physical and some biological properties of Product R are described in U.S. Patent Nos. 6,303,153 and 6,528,098.

Product R has been used as a synonym of Reticulose<(R)> in some literature. For the purpose of the present application, Product R (AVR118) and Reticulose<(R)> represent two distinct products.

Product R is a therapeutic composition for treating viral infections and stimulating the immune system comprising nucleotides and peptides have molecular weights not more than 14 KDa and substantially not more than 8 KDa, wherein said nucleotides and peptides are breakdown products of casein, peptone, RNA and serum albumin, and wherein said composition has a light absorption spectrum with typical absorption ratios of 2.0 (+-10%) at 260 nm/280 nm and 1.4 (+-10%) at 260 nm/230 nm.

Generally, Product R is prepared according to the following manner.

First, the starting materials casein, beef peptone, RNA, bovine serum albumin (BSA), and sodium hydroxide are suspended in proportions of, by weight, 35-50% (casein), 15-40% (beef peptone), 10-25% (RNA), 1-10% (BSA) and 5-25% (sodium hydroxide) in an appropriate volume of distilled water. All starting materials are generally available or otherwise can be readily prepared by a person of ordinary skill in the art. While any RNA is suitable for the intended purpose of the present invention, plant RNA is preferred and yeast RNA is the most preferred. The ratio of total proteins versus the volume of distilled water is generally about 1.5-2.5 to about 100 by weight, preferably about 2.2 to about 100 by weight. This means that every 1.5-2.5 grams of the total proteins are suspended in about 100 milliliters of distilled water.

The suspension as prepared above is then autoclaved at a pressure of approximately 5-15 lbs., preferably 8-10 lbs. under an elevated temperature in a range, for example, from about 65[deg.]-I 50<0>C, preferably from about 90[deg.] -110<0>C, over a period of approximately 2-10 hours, preferably more than 3 hours. As known to a person of ordinary skill in the art, under such conditions RNA may be completely hydrolyzed into nucleotides. After autoclaving, the solution is cooled down to room temperature, and then allowed to stay at a temperature of 3[deg.] to 8<0>C for at least 12 hours to precipitate insoluble elements. Alternatively, the cooled solution may be centrifuged at a temperature below 8<0>C to remove the precipitates.

The resulting solution is then filtered through a 2 micron and a 0.45 micron filters under an inert gas such as nitrogen or argon at a pressure of about 1-6 psi. In a similar manner the solution is filtered again through a pyrogen retention filter, preferably 0.2 micron.

After the above filtration, the solution may be cooled at 3 to 8<0>C again for at least about 12 hours and filtered again in the same way as described above.

The resulting filtrate is then assayed for total nitrogen content using methods known to a person of ordinary skill in the art such as Kjeldahl method (Kjeldahl, Z. 1983, Anal. Chem., Vol. 22:366), and its improvements. Based on the assay, the filtrate is then diluted with chilled distilled water to an appropriate volume having a preferred total nitrogen content ranging from 165 to 210 mg/ml.

The pH of the diluted solution is then adjusted with HCl to a physiologically acceptable pH, preferably to about 7.3 to 7.6, after which the diluted solution is filtered again through a 0.2 micron filter under an inert gas as described above.

Product R so produced contains essentially nucleotides, nucleosides and free nucleic acid bases of low molecular weights from a complete hydrolysis of RNA and small peptides from partial hydrolysis of the proteins. It is possible that the base hydrolysis of the proteins also produces free amino acids.

It is understood that the use of a filtration technique is essentially to remove bacteria or other particles having similar size to or larger size than bacteria. Thus, any filter regardless of its manufacturer or material from which it is made is suitable for the intended purpose. All filters used in the present process are widely available to a person of ordinary skill in the art.

The final filtrate is then filled and sealed into appropriate vials, such as 2 ml or 10 ml glass vials under an inert gas. The filled vials are autoclaved for final sterilization, after which they are ready for use.

An analysis of the composition of Product R reveals that Product R contains two major components, which are exhibited as two bands having molecular weights of 5.2 kDa and 4.3 kDa on a SDS-polyacrymide gel electrophoresis, namely peptide-A and peptide B, respectively. Peptide A is a 31 amino acid long peptide of 5.2 KDa molecular weight derived from bovine casein. It is a straight chain peptide lacking any cysteine- cysteine cross links. The structure is remarkable for six proline residues spaced throughout the molecule and four basic glutamine residues. Since proline residues can induce bends in peptide chains, the structure is likely a highly folded peptide. Peptide-B comprises a 21 amino acid long linear peptide of 4.3 KDa molecular weight covalently linked at the hydroxyl group of a serine residue at position 18 to a diadenosine dinucleotide unit through a diphosphodiester linkage at the 3 '-position. Peptide-A and peptide-B are present in the Product R composition in an approximately equal amount and the total amount of these two peptides is about 4.8-5.3 mg/ml, determined by a Lowry protein assay. In certain embodiments of the invention, peptide A, peptide B (as a peptide-nucleic acid conjugate), or a combination of peptide A and peptide B can be administered to a patient to provide palliative care according to the invention (e.g., where the patient has not and does not have chemotherapy or radiation therapy). Pharmaceutical compositions comprising peptide A, peptide B (as a peptide-nucleic acid conjugate), or a combination of peptide A and peptide B with another medicament are also provided.

The sequence of peptide A is:
KVLPVPQKAVPYPQRDMPIQAFLLYQEPVLG (SEQ ID NO. 1). The sequence of peptide B is: GEIPD AGGRTVD YYVGFSDSV (SEQ ID NO. 2). Product R also comprises nucleosides, nucleoside diphosphates and nucleoside monophosphates. There are sixteen identified constituent compounds present in the Product R formulation - 3 nucleosides, two nucleoside diphosphates and eight nucleoside monophosphates, together with two peptides (one of them a peptide-nucleic acid conjugate) and sodium chloride.

The physical, chemical and biological properties of Product R are further described in U.S. Patent Nos. 6,303,153 and 6,528,098.

### 4.1. Target Diseases

The methods of the present invention are applicable for treating any type of disease, particularly, end-stage or terminal disease to improve the quality of life. The methods of the present invention are also applicable for treating or preventing conditions, such as cachexia, anorexia, weakness and fatigue, in a patient, preferably a patient suffering from end-stage disease. End-stage disease is the last phase in the course of a progressive disease. The only "stage" past "end stage" is usually death or a reprieve from it by a transplant in certain conditions. A terminal or end-stage cancer is a disease that cannot be cured and will cause death.

There are numerous disorders in which palliative care would be beneficial, such as, for example, cancer, congestive heart failure, infections (e.g., those causing chronic viral illnesses), chronic obstructive pulmonary disease, end-stage renal disease, autoimmune disorders (e.g., rheumatoid arthritis), multiple sclerosis and other neurological disorders, etc. Preferably, the cancer is a non-lymphocytic cancer. Patients with hematological cancers generally do not have cachexia (Inui, 2002, CA Cancer J. Cllin 52:72-91). In another embodiment, the cancer is not a basal cell carcinoma. Representative non- lymphocytic cancers, include, but are not limited to, anal cancer, bile duct cancer, bladder cancer, brain cancer, breast cancer, cervical cancer, colon cancer, endometrial cancer, esophageal cancer, gall bladder cancer, gastrointestinal cancer, head and neck cancer, Kaposi's sarcoma, kidney cancer, laryngeal cancer, liver cancer, lung cancer, melanoma, mesothelioma, oral cavity cancer, ovarian cancer, pancreatic cancer, penile cancer, prostate cancer, rectal cancer, sarcoma, testicular cancer, thymomas, thyroid cancer, or uterine cancer. In patients to be treated with the methods of the invention, the cancer has generally metastesized. Cancers to be treated with the methods of the invention are described in De Vita et al. (Eds), CANCER: Principles and Practice of Oncology, 6th edition, Lippincott Williams and Wilkins, 2001.

In the methods of the invention, the patient is not undergoing chemotherapy. In a preferred embodiment, the patient is not a candidate for chemotherapy and/or does not wish to undergo chemotherapy. For example, a patient treatable with methods of the invention may have stopped responding to chemotherapy, may not be able to tolerate chemotherapy or may be allergic to chemotherapy. In other embodiments, the patient has ceased chemotherapy, preferably has not had chemotherapy for 2 weeks or more, 3 weeks or more, 4 weeks or more, or longer. In yet other embodiments, the patient has not had chemotherapy within 2 weeks, within 3 weeks, within 4 weeks, within 6 weeks, or within 8 weeks. In a most preferred embodiment, the patient has ceased chemotherapy for 3 weeks or more before treatment with Product R. In another embodiment, the patient has less than 10%, 5%, 1%, 0.5%, or 0.1% of a chemotherapeutic present as determined by the half-life of the chemotherapeutic or as measured by standard assays. In certain embodiments, the patient is not undergoing radiation therapy.

### 4.2. Dosage and Administration

The individual, patient or subject in whom palliative care, or treatment of cachexia, loss of appetite or fatigue, is desired is an animal, preferably a mammal, a non- human animal or primate, and most preferably human. The term "animal" as used herein includes, but is not limited to, companion animals, such as cats and dogs; zoo animals; wild animals, including deer, foxes and raccoons; farm animals, livestock and fowl, including horses, cattle, sheep, pigs, turkeys, ducks, and chickens, as well as any rodents.

The methods of the invention comprise administering an amount of Product R effective to provide palliative care, or treat cachexia, loss of appetite or fatigue. An amount of Product R effective to provide palliative care, or treat cachexia, loss of appetite or fatigue, within the meaning of the present invention can be determined by a patient's attending physician or veterinarian. Such amounts are readily ascertained by one of ordinary skill in the art and can provide palliative care when administered in accordance with the present invention. Factors which influence what an amount of Product R effective to provide palliative care, or treat cachexia, loss of appetite or fatigue, will be include, the absence or presence of infection, and the age, physical condition, existence of other disease states, and nutritional status of the patient. Additionally, other medications the patient may be receiving will effect the determination of the amount of Product R to administer. In certain embodiments, the amount of Product R effective to provide palliative care, or treat cachexia, loss of appetite or fatigue, is in a range from about 0.5 microliters to about 100 microliters per kilogram of body weight per day, or from about 2.5 microliters to about 40 microliters per kilogram of body weight per day, or from about 10 microliters to about 25 microliters per kilogram of body weight per day. In another embodiment, the amount of Product R is in a range from about 40 microliters to about 20 milliliters per day, or from about 100 microliters to about 10 milliliters per day, or from about 0.4 milliliters to about 4.0 milliliters per day. As used herein, "about" entails normal experimental variation. Alternatively, Product R may be administered to the patient according to the conventional doses or any dosages that are apparent to a person of ordinary skill in the art.

The desired dose may be administered once a day or in two, three or more sub-doses at appropriate intervals, generally equally spread in time, throughout the day.

Typical routes of administration for Product R may include, without limitation, oral, topical, parenteral, sublingual, rectal, vaginal, ocular, and intranasal. Parenteral administration includes subcutaneous injections, intravenous, intramuscular, intraperitoneal, intrapleural, intrasternal injection or infusion techniques. In a preferred embodiment of the invention, Product R is administered systemically, by the subcutaneous route. It will be appreciated that the preferred route may vary with, for example, the condition and age of the recipient.

The present invention also encompasses methods for providing palliative care in a patient, comprising administering to said patient an amount of Product R effective to treat or prevent cachexia and an effective amount of another medicament. In certain embodiments, the other medicament is an analgesic. Pharmacological agents for treating cancer pain are summarized in Bruera et ah, 1998, CMAJ 158:1717-26. Analgesics useful in the methods of the invention include, but are not limited to, nonopioid analgesics, such as acetaminophen and nonsteroidal anti-inflammatory drugs (NSAIDs); opioid analgesics, such as codeine, hydromorphone, morphine, oxycodone, fentanyl, and methadone. Adjuvant drugs useful in the methods of the invention include, but are not limited to, tricyclic antidepressants, corticosterioids, anticonvulsants, biphosphonates, oral local anesthetics, pyschostimulants and muscle relaxants. In other embodiments, the other medicament is a vitamin {e.g., Vitamins A, C, D, E, K, and the eight B vitamins-biotin, folate, niacin, pantothenic acid, riboflavin, thiamin, vitamin B6, and vitamin B 12), an herbal supplement {e.g., chondroitin [see McKenna, Botanical Medicines: The Desk Reference for Major Herbal Supplements, Haworth Herbal Press (2002)]), a sleep aid {e.g., Zolpidem Tartrate (Ambien<(R)>)), a lipid lowering agent {e.g., Atorvastatin Calcium (Lipitor<(R)>)), an anti-asthmatic agent {e.g., theophylline), an anti-constipation agent {e.g., Senokot<(R)> (Purdue Products L.P., Stamford, Connecticut)), an anti- heartburn agent {e.g., Nexium, Pepcid, Prevacid, Prilosec, Tagamet, Zantac, etc.), or an anti-hypertensive agent. Other examples of these medicaments can be found in, for example, Physician's Desk Reference, 59 edition, Thomson (2003) and the Merck Index, 13th edition, Merck Publishing (2001).

As used herein, the term "in combination" refers to the use of more than one prophylactic and/or therapeutic agents. The use of the term "in combination" does not restrict the order in which prophylactic and/or therapeutic agents are administered to a subject with a disorder. A first prophylactic or therapeutic agent can be administered prior to {e.g., up to 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to {e.g., up to 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second prophylactic or therapeutic agent to a subject which had, has, or is susceptible to a wound. The prophylactic or therapeutic agents are administered to a subject in a sequence and within a time interval such that the agent of the invention can act together with the other agent to provide an increased benefit than if they were administered otherwise. Any additional prophylactic or therapeutic agent can be administered in any order with the other additional prophylactic or therapeutic agents.

### 4.3 Monitoring Effects During Therapy

The effects/efficacy of treatment of an end-stage disease according to the present invention can be detected, for example, on the level of the molecular and cellular agents involved in the immune response (e.g., TNF-[alpha]) or in a patient by measuring, e.g., body weight, body mass index (BMI), percent fat, strength, calf and arm circumference, skin fold measurement using standard methods well known to one of ordinary skill in the art. In certain embodiments, the methods of the invention results in an increase of 4%, 5%, 10%, 15%, 20%, 25% or more in one or more of the above parameters.

Quality of life (QOL) parameters can also be measured including, but not limited to, mood, appetite, alertness, activities of daily living (ADLs), malaise, myalgia and power/energy using standard methods well known to one of ordinary skill in the art. Useful QOL measures include, but are not limited to, the Karnovsky index (Mor et at, 1984, Cancer 53:2002-2007), the EORTC QLQ-30 Questionaire (version 3) by the European Organisation for Research and Treatment of Cancer, the Mini-Mental State Examination by Psychological Assessment Resources, Inc., the Brief Fatigue Inventory by the M.D. Anderson Cancer Center, and the Memorial Pain Scale by Memorial Sloan Kettering Hospital. QOL questionnaires used in advanced lung cancer are described in Gralla, 2004, The Oncologist 9(suppl 6): 14-24. In certain embodiments, the methods of the invention results in an increase of 10, 20 or more in the QOL score as measured by the Karnovsky index.

### 4.4. Pharmaceutical Formulations

While it is possible for Product R to be administered as part of a pharmaceutical formulation, it is preferable to present it alone, although it may be administered at about the same time as one or more other pharmaceuticals are independently administered. If Product R is administered as part of a pharmaceutical formulation, the formulations of the present invention comprise at least one administered ingredient, i.e., Product R, as above defined, together with one or more acceptable carriers thereof and optionally one or more additional medicaments. Suitable additional medicaments are provided in Section 4.2. The carrier(s) must be "pharmaceutically acceptable" in the sense of being compatible with the other ingredients of the formulation and not deleterious to the recipient thereof (e.g., suitable for in vivo use, in the patient). Preferably, Product R in a pharmaceutical formulation is sterile.

The formulations may conveniently be presented in unit-dose or multi-dose containers, e.g., sealed ampules and vials. Preferred unit dosage formulations are those containing a daily dose or unit, daily sub-dose, or an appropriate fraction of the administered ingredient.

The compositions of the invention can be in the form of a solid, liquid or gas (aerosol). Pharmaceutical compositions of the invention can be formulated so as to allow a compound of the invention to be bioavailable upon administration of the composition to a subject. Compositions can take the form of one or more dosage units, where for example, a tablet can be a single dosage unit, and a container of a compound of the invention in aerosol form can hold a plurality of dosage units. A syringe containing a unit dose of Product R is also provided.

For oral administration, the pharmaceutical preparation can be in liquid form, for example, solutions, syrups or suspensions, or can be presented as a drug product for reconstitution with water or other suitable vehicle before use. Such liquid preparations can be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, or fractionated vegetable oils); and preservatives (e.g., methyl or propyl-p- hydroxybenzoates or sorbic acid). The pharmaceutical compositions can take the form of, for example, tablets or capsules prepared by conventional means with pharmaceutically acceptable excipients such as binding agents (e.g., pregelatinized maize starch, polyvinyl pyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets can be coated by methods well-known in the art.

Preparations for oral administration can be suitably formulated to give controlled release of the active compound.

For buccal administration, the compositions can take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g. , dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, e.g., gelatin for use in an inhaler or insufflator can be formulated containing a powder mix of the compound and a suitable powder base such as lactose or starch.

The compounds can be formulated for parenteral administration by injection, e.g., by bolus injection or continuous infusion. Formulations for injection can be presented in unit dosage form, e.g., in ampoules or in multi-dose containers, with an added preservative. The compositions can take such forms as suspensions, solutions or emulsions in oily or aqueous vehicles, and can contain formulatory agents such as suspending, stabilizing and/or dispersing agents. Alternatively, the active ingredient can be in powder form for constitution with a suitable vehicle, e.g., sterile pyrogen-free water, before use.

The compounds can be formulated in compositions such as creams, lotions, gels, opthalmic drops, ointments, solutions, suspensions, shampoos, or other forms known to one of skill in the art and described in, for example, Remington's Pharmaceutical Sciences,' 16th and 18th eds., Mack Publishing, Easton Pa. (1980 & 1990), and Introduction to Pharmaceutical Dosage Forms, 4th ed., Lea & Febiger, Philadelphia (1985). Actual methods for preparing pharmaceutical compositions are known or apparent to those skilled in the art and are described in detail in, for example, Remington's Pharmaceutical Sciences, 16th and 18th eds., Mack Publishing, Easton Pa. (1980 & 1990).

The compounds can also be formulated in rectal compositions such as suppositories or retention enemas, e.g., containing conventional suppository bases such as cocoa butter or other glycerides.

In addition to the formulations described previously, the compounds can also be formulated as a depot preparation. Such long acting formulations can be administered by implantation (e.g., subcutaneously or intramuscularly) or by intramuscular injection. Thus, for example, the compounds can be formulated with suitable polymeric or hydrophobic materials (e.g., as an emulsion in an acceptable oil) or ion exchange resins, or as sparingly soluble derivatives, for example, as a sparingly soluble salt. Liposomes and emulsions are well known examples of delivery vehicles or carriers for hydrophilic drugs.

### 4.5. Kits

The invention also provides kits for carrying out the methods and/or therapeutic regimens of the invention. In one embodiment, such kits comprise in one or more containers, Product R. In another embodiment, such kits comprise in one or more containers an amount of Product R effective to provide palliative care, or treat loss of appetite or fatigue, in pharmaceutically acceptable form.

Product R in a container of a kit of the invention may be in the form of a pharmaceutically acceptable solution, e.g., in combination with sterile saline, dextrose solution, or buffered solution, or other pharmaceutically acceptable sterile fluid. Alternatively, Product R may be lyophilized or desiccated; in this instance, the kit optionally further comprises in a container a pharmaceutically acceptable solution {e.g., saline, dextrose solution, etc.), preferably sterile, to reconstitute Product R to form a solution for injection purposes.

In another embodiment, a kit of the invention further comprises a needle or syringe, preferably packaged in sterile form, for injecting Product R, and/or a packaged alcohol pad. Instructions are optionally included for administration of Product R by a clinician or by the patient.

Kits are also provided for carrying out the combination therapies of the present invention. In one embodiment, a kit comprises a first container containing Product R and a second container containing an additional medicament. Suitable additional medicaments are provided in Section 4.2.

The kit may for example comprise metal or plastic foil, such as a blister pack. The kit may be accompanied by one or more reusable or disposable device(s) for administration (e.g, syringes, needles, dispensing pens) and/or instructions for administration.

The following examples only serve to further illustrate, but not to limit the scope of the present invention.

### 5. EXAMPLES

### 5.1. Example 1: Method for Preparing Product R

About 35.0 g of casein, about 17.1 g of beef peptone, about 22.0 g of nucleic acid (RNA), about 3.25 g bovine serum albumin were suspended in about 2.5 liters of water for injection USP at about 3 to 7<0>C in a suitable container and gently stirred until all the ingredients have been properly wet. About 16.5 g of sodium hydroxide (reagent grade ACS) is added with stirring. Stirring is continued until the sodium hydroxide is completely dissolved. The reaction is autoclaved at about 9 Ibs pressure and 200-230 <0>F for a period of time until RNA is completely digested, for example, about 4 hours. At the end of the period, the autoclave was stopped and the reaction flask and contents were permitted to slowly cool to ambient temperature. The reaction was then cooled for at least six hours at about 3-8[deg.] C. The resulting solution was filtered through 2 micron and 0.45 micron filters using an inert gas such as nitrogen or argon at low pressure (1-6 psi). In a similar manner, the solution was filtered again through 0.2 micron pyrogen retention filters. The resulting filtrate was sampled and assayed for total nitrogen. A calculation was then performed to determine the quantity of cooled water for injection to be added to the filtrate to yield a diluted filtrate with a nitrogen content between about 165-210 mg/100 ml, the final volume is approximately 5 liters. The pH was then adjusted with either concentrated HCl (reagent grade ACS) or 1.0 normal NaOH to about 7.3-7.6. The diluted solution was then filtered again through 0.2 micron filters with inert gas at low pressure. The final filtrate was then filled and sealed into 2 ml glass ampoules or 2mL vials while in an inert gas atmosphere. The ampoules or vials were collected and autoclaved for final sterilization at 240[deg.] F and 20 to 30 psi pressure for about 30 minutes. Following the sterilization cycle, the ampules with Product R were cooled and washed.

All quantities are subject to plus or minus 2.5% variation for pH, volume, and analytical adjustments.

### 5.2. Example 2

A phase 1 clinical trial was conducted. Two cachectic patients with advanced stage solid tumors for whom no curative or life-prolonging therapy was available, or whom had declined anti-neoplastic therapy were enrolled in the trials. These two patients had advanced pancreatic cancer and received a single dose of Product R subcutaneously at a dose of 0.4 ml/day. The patients were observed for one week, and, if no Grade 3 or 4 adverse events occurred, were to be given an additional 24 doses for 28 days (6 days/week). Patients were followed for 28 days after treatment was completed. Total weight, body mass index (BMI), fat percentage, strength, calf and arm circumference, and skin fold were measured. Spontaneous patient comments regarding quality of life (QOL) parameters, including improvement in mood, appetite, alertness, activities of daily living (ADLs), malaise, myalgia and power/energy were recorded using the Kanofsky Score (KS). Adverse events were monitored. Both patients were on pain medication and treatment for GI complications of their pancreatic cancer.

The Kanofsky Scores were well maintained during treatment with Product R. After Product R was discontinued, the Kanofsky Scores decreased.

Highlights of the effect of Product R in both patients include the following:

Improved weight (>4% increase)

Improved fat percentage (20% increase)

Improved strength (5 % increase)

The effects on weight and fat percentage were maintained after discontinuation of treatment.

Hematology and chemistry values remained stable throughout the treatment and follow-up periods. There did not appear to be any trends in changes of cytokine levels.

Conclusion:

Patients treated with Product R at a 0.4mg/day dose demonstrated improvements in weight gain and fat percentage which continued after discontinuation of the drug. Patients undergoing treatment with Product R maintained their quality of life, but their quality of life decreased after discontinuation of the drug.

### 5.3. Example 3

A phase II double blind, placebo controlled, randomized, multi-center study with Product R solution in patients with advanced malignancies who are not candidates for chemotherapy is being undertaken. The purpose of the study is to explore the effect of Product R Solution given subcutaneously on weight, appetite, performance status, and other measures of quality of life related to advanced malignancies as compared to patients not receiving Product R Solution.

Approximately 40 patients with histologically confirmed advanced non- small cell lung cancer, pancreatic cancer, cervical cancer, sarcoma, lymphoma or leukemia, and multiple myeloma, who are not candidates for, or who refuse, chemotherapy are to be enrolled. Eligible patients will be between the ages of 18 and 80, will have a Karnofsky performance score <80 and > 40%, and a life expectancy of >4 months. A complete medical history will be documented, including the patient's cancer history. A complete physical examination will be conducted, including measurement of vital signs, body weight and height, lean body mass, and a anteroposterior and lateral chest x-ray and 12-lead electrocardiogram will be performed.

Disease-related symptoms will be assessed, and a Mini-mental status examination and Karnofsky performance status score will be determined. The Brief Fatigue Inventory and a QOL assessment will be performed. Laboratory samples will be collected for hematology, electrolytes, clinical chemistry, T3, T4, TSH and urinalysis as well as for cytokine levels.

Product R will be given subcutaneously each day indefinitely for as long the physician or caregiver feels that there is a benefit to the patient. Patients will receive daily doses of 4.0 mL Product R subcutaneously.

Safety and efficacy evaluations will be performed. Brief examinations will be conducted weekly including measurement of vital signs, body weight and lean body mass. Symptom directed or complete physical examinations will be performed during the study as clinically indicated. Disease-related symptoms will be assessed, and mental status, Karnofsky performance status score, fatigue and general quality of life will be determined. Laboratory samples will be collected for hematology, electrolyte, clinical chemistry, urinalysis parameters and cytokines. In addition, adverse events and all concomitant medications and procedures and supportive therapies will be documented.

## Claims

1. A pharmaceutical composition comprising
a) an amount of Product R in the range from about 0.5 microliters to about 100 microliters per kilogram of body weight per day effective to treat or prevent cachexia, and
b) a pharmaceutically acceptable carrier wherein said Product R comprises nucleotides and peptides having molecular weights not more than 14 KDa, wherein said nucleotides and peptides are breakdown products of casein, peptone, RNA and serum albumin, and wherein said composition has a light absorption spectrum with typical absorption ratios of 2.0 (±10%) at 260 nm/280 nm and 1.4 (±10%) at 260 nm/230 nm for use in a method of providing palliative care to a patient in need thereof comprising administering to said patient an amount of Product R effective to treat or prevent cachexia or to improve quality of life (QOL), wherein said patient has a non-lymphocytic cancer which is not a basal cell carcinoma, wherein within three weeks of said administering of said Product R said patient has not and does not have chemotherapy or radiation therapy.

2. The composition of claim1, wherein the patient is human.

3. The composition of claim 1, wherein the amount of Product R is In a range from about 2.5 microliters to about 40 microliters per kilogram of body weight per day, or wherein the amount of Product R is in a range from about 10 microliters to about 25 microliters per kilogram of body weight per day.

4. The composition of claim 1, wherein the Product R is administered parenterally, topically or systemically.

5. The composition of claim 1, wherein the cancer is anal cancer, bile duct cancer, bladder cancer, brain cancer, breast cancer, cervical cancer, colon cancer, endometrial cancer, esophageal cancer, gall bladder cancer, gastrointestinal cancer, head and neck cancer, kidney cancer, Kaposi's sarcoma, laryngeal cancer, liver cancer, lung cancer, melanoma, mesothelioma, oral cavity cancer, ovarian cancer, pancreatic cancer, penile cancer, prostate cancer, rectal cancer, sarcoma, testicular cancer, thymomas, thyroid cancer, or uterine cancer.

6. The composition of claim 1, further comprising administering to the patient an effective amount of an analgesic, preferably wherein the analgesic is acetaminophen, a nonsteroidal anti-inflammafiory drug, codeine, hydromorphone, morphine, oxycodone, fentanyl, or methadone.

7. The composition of claim 1, further comprising administering to the patient an effective amount of an adjuvant drug, preferably wherein the adjuvant drug is a tricyclic antidepressant, corticosteroid, anticonvulsant, biphosphonate, oral local anaesthetic, pyschostimulant or muscle relaxant.

8. The composition of claim 1, further comprising administering to the patient an effective amount of a vitamin, an herbal supplement, a sleep aid, a lipid lowering agent, an anti-asthmatic agent, an anti-constipation agent, an anti-heartbum agent, or an antihypertensive agent.

## Patentansprüche

1. Pharmazeutische Zusammensetzung, enthaltend
a) eine Menge an Produkt R im Bereich von etwa 0,5 Mikroliter bis etwa 100 Mikroliter pro Kilogramm Körpergewicht pro Tag, ausreichend für die Behandlung bzw. Prävention von Kachexie, und
b) einen pharmazeutisch unbedenklichen Träger, wobei das Produkt R Nukleotide und Peptide mit Molekulargewichten von nicht mehr als 14 kDA umfasst, wobei es sich bei den Nukleotiden und Peptiden um Abbauprodukte von Kasein, Pepton, RNA und Serumalbumin handelt, und wobei die Zusammensetzung ei n Lichtabsorptionsspektrum mit typischen Absorptionsverhältnissen von 2,0 (±10%) bei 260 nm/280 nm und 1,4 (±10%) bei 260 nm/230 nm aufweist, zur Verwendung in einem Verfahren zur Bereitstellung von palliativer Pflege bei einem derer bedürftigen Patienten, bei dem man dem Patienten eine für die Behandlung oder Prävention von Kachexie oder die Verbesserung der Lebensqualität (Quality of Life, QOL) wirksamen Menge an Produkt R verabreicht, wobei der Patient an nicht lymphozytischem Krebs leidet, bei dem es sich nicht um ein Basalzellkarzinom handelt, wobei sich der Patient innerhalb von drei Wochen nach de r Verabreichung de s Produkts R keiner Chemotherapie oder Strahlentherapie unterzieht.

2. Zusammensetzung nach Anspruch 1, wobei es sich bei dem Patienten um einen Menschen handelt.

3. Zusammensetzung nach Anspruch 1, wobei die Menge an Produkt R im Bereich von etwa 2,5 Mikroliter bis etwa 40 Mikroliter pro Kilogramm Körpergewicht pro Tag liegt, oder wobei die Menge an Produkt R im Bereich von etwa 10 Mikroliter bis etwa 25 Mikroliter pro Kilogramm Körpergewicht pro Tag liegt.

4. Zusammensetzung nach Anspruch 1, wobei das Produkt R parenteral, topisch oder systemisch verabreicht wird.

5. Zusammensetzung nach Anspruch 1, wobei es sich bei dem Krebs um Analkrebs, Gallengangskrebs, Blasenkrebs, Hirnkrebs, Brustkrebs, Gebärmutterhalskrebs, Dickdarmkrebs, Endometriumkrebs, Speiseröhrenkrebs, Gallenblasenkrebs, gastrointestinalen Krebs, Kopf- und Halskrebs, Nierenkrebs, Kaposi-Sarkom, Kehlkopfkrebs, Leberkrebs, Lungenkrebs, ein Melanom, ein Mesotheliom, einen Krebs der Mundhöhle, Eierstockkrebs, Bauchspeicheldrüsenkrebs, Peniskrebs, Prostatakrebs, Rektalkrebs, ei n Sarkom, Hodenkrebs, ei n Thymom, Schilddrüsenkrebs oder Gebärmutterkrebs handelt.

6. Zusammensetzungen nach Anspruch 1, bei der man weiterhin dem Patienten eine wirksame Menge eines Analgetikums verabreicht, wobei es sich bei dem Analgetikum vorzugsweise um Acetaminophen, ein nichtsteroidales antiinflammatorisches Arzneimittel, Codein, Hydromorphon, Morphin, Oxycodon, Fentanyl oder Methadon handelt.

7. Zusammensetzung nach Anspruch 1, bei der man dem Patienten weiterhin eine wirksame Menge eines Adjuvans-Arzneimittels verabreicht, wobei es sich bei dem Adjuvans-Arzneimittel vorzugsweise um ein tricyclisches Antidepressivum, ein Corticosteroid, ein Antikonvulsivum, ein Biphosphonat, ein orales Lokalanästhetikum, ei n Psychostimulanz oder ein muskelrelaxierendes Mittel handelt.

8. Zusammensetzung nach Anspruch 1, wobei man dem Patienten weiterhin eine wirksame Menge eines Vitamins, eines pflanzlichen Zusatzstoffes, eines schlaffördernden Mittels, eines lipidsenkenden Mittels, eines Antiasthmatikums, eines Mittels gegen Verstopfung, eines Mittels gegen Sodbrennen oder eines blutdrucksenkenden Mittels verabreicht.

## Revendications

1. Composition pharmaceutique comprenant :
a) une quantité de Produit R comprise entre environ 0 , 5 microlitre et environ 100 microlitres par kilogramme de masse corporelle par jour, permettant de traiter ou de prévenir efficacement la cachexie, et
b) un vecteur de qualité pharmaceutique où ledit Produit R est constitué de nucléotides et de peptides de masse moléculaire ne dépassant pas 14 kDa, lesdits nucléotides et peptides étant des produits de fragmentation de la caséine, de la peptone, de l'ARN et de l'albumine sérique, et ladite composition présentant un spectre d'absorption de la lumière de rapport d'absorption typique égal à 2,0 (± 10 %) à 260 nm/280 nm et à 1,4 (± 10 %) à 260 nm/230 nm, pour emploi dans une méthode de soins palliatifs à destination d'un patient les nécessitant, ladite méthode comprenant l'administration audit patient d'une quantité de Produit R permettant de traiter ou de prévenir efficacement la cachexie ou d'améliorer la qualité de vie (QDV), ledit patient souffrant d'un cancer non lymphocytaire qui n'est pas un carcinome à cellules basales, de sorte que dans les trois semaines qui suivent l'administration dudit Produit R, ledit patient ne subisse pas de chimiothérapie ni de radiothérapie.

2. Composition conforme à la revendication 1, où le patient est humain.

3. Composition conforme à la revendication 1, où la quantité de Produit R est comprise entre environ 2,5 microlitres et environ 40 microlitres par kilogramme de poids corporel par jour, ou où la quantité de Produit R est comprise entre environ 10 microlitres et environ 25 microlitres par kilogramme de poids corporel par jour.

4. Composition conforme à la revendication 1, où le Produit R est administré par voie parentérale, topique ou systémique.

5. Composition conforme à la revendication 1, où le cancer est l'un des suivants : cancer de l'anus, cancer des voies biliaires, cancer de la vessie, cancer du cerveau, cancer du sein, cancer du col de l'utérus, cancer du côlon, cancer de l'endométrium, cancer de l'oesophage, cancer de la vésicule biliaire, cancer gastro-intestinal, cancer de la tête et du cou, cancer du rein, sarcome de Kaposi, cancer du larynx, cancer du foie, cancer du poumon, mélanome, mésothéliome, cancer de la cavité buccale, cancer des ovaires, cancer du pancréas, cancer du pénis, cancer de la prostate, cancer du rectum, sarcome, cancer du testicule, thymome, cancer de la thyroïde ou cancer de l'utérus.

6. Composition conforme à la revendication 1, comprenant en outre l'administration au patient d'une quantité active d'un analgésique, l'analgésique étant préférentiellement l'un des suivants : acétaminophène, anti-inflammatoire non stéroïdien, codéine, hydromorphone, morphine, oxycodone, fentanyl ou méthadone.

7. Composition conforme à la revendication 1, comprenant en outre l'administration au patient d'une quantité active d'un médicament adjuvant, le médicament adjuvant étant préférentiellement l'un des suivants : antidépresseur tricyclique, corticostéroïde, antiépileptique, biphosphonate, anesthésique oral local, psychostimulant ou myorelaxant.

8. Composition conforme à la revendication 1, comprenant en outre l'administration au patient d'une quantité active d'une vitamine, d'un supplément végétal, d'un somnifère, d'un agent diminuant la lipidémie, d'un agent antiasthmatique, d'un agent anti-constipation, d'un agent anti-pyrosis ou d'un agent antihypertenseur.
